(19) Europäisches Patentamt / European Patent Office / Office européen des brevets

(11) **EP 3 078 968 A1**

(12) **EUROPEAN PATENT APPLICATION**

(43) Date of publication:
**12.10.2016 Bulletin 2016/41**

(21) Application number: **16159345.4**

(22) Date of filing: **09.03.2016**

(51) Int Cl.:
*G01N 29/04* (2006.01)    *G01N 29/44* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **11.03.2015 JP 2015048471**

(71) Applicant: **Seiko Epson Corporation**
**Shinjuku-ku**
**Tokyo 163-0811 (JP)**

(72) Inventor: **Kano, Kazuyuki**
**Nagano 392-8502 (JP)**

(74) Representative: **Hoffmann Eitle**
**Patent- und Rechtsanwälte PartmbB**
**Arabellastraße 30**
**81925 München (DE)**

(54) **ULTRASONIC APPARATUS, CONTROL DEVICE FOR ULTRASONIC APPARATUS, PROBE, AND PEELING DETECTION METHOD**

(57)    A control device for an ultrasonic apparatus includes: a reference value setting section that sets a reference value based on either the reflectance of an ultrasonic wave reflected from the interface with the air or the reflectance of an ultrasonic wave reflected from the interface with a measurement target; a reference period setting section that sets a reference period ended at the earlier time than the time of receiving an ultrasonic wave that is transmitted from an ultrasonic transducer element and is reflected from the measurement target; and a peeling detecting section that detects peeling of the ultrasonic transducer element by comparing a reception signal, which is output from the ultrasonic transducer element in the reference period, with the reference value.

**Description**

BACKGROUND

1. Technical Field

The present invention relates to an ultrasonic apparatus and a control device for an ultrasonic apparatus.

2. Related Art

JP-A-2013-55978 discloses an ultrasonic transducer. Not only an ultrasonic transducer element group of a transmission unit and an ultrasonic transducer element group of a receiving unit but also an ultrasonic transducer element group for peeling detection are disposed on a sensor substrate. These ultrasonic transducer element groups are covered by an adhesive layer. The adhesive layer is in close contact with the body when generating an ultrasonic image. As a result, ultrasonic waves output from the transmission unit are transmitted from the adhesive layer to the body without passing through the air layer, and are reflected by organs, such as blood vessels or internal organs, to be transmitted again from the body to the adhesive layer.

In JP-A-2013-55978, a time from transmission to reception of the ultrasonic wave is measured upon determination of peeling. The measured time is compared with an adhesive layer surface reflection time measured in advance. The adhesive layer surface reflection time corresponds to the propagation time of the ultrasonic wave reflected from the surface of the adhesive layer, that is, an interface with the air. If the ultrasonic transducer element is not peeled off from the body, the ultrasonic wave is transmitted through the surface of the adhesive layer and reaches an organ in the body. Accordingly, a longer time is required until the reflected wave is received by the ultrasonic transducer element than that in a case in which the ultrasonic wave is reflected from the surface of the adhesive layer. As a result, the processing time is increased by the amount upon determination of peeling.

SUMMARY

An advantage of some aspects of the invention is to provide a control device for an ultrasonic apparatus that can shorten the processing time upon determination of peeling.

(1) An aspect of the invention relates to a control device for an ultrasonic apparatus including: a reference value setting section that sets a reference value based on either a reflectance of an ultrasonic wave reflected from an interface with the air or a reflectance of an ultrasonic wave reflected from an interface with a measurement target; a reference period setting section that sets a reference period ended at an earlier time than a time of receiving an ultrasonic wave that is transmitted from an ultrasonic transducer element and is reflected from the measurement target; and a peeling detecting section that detects peeling of the ultrasonic transducer element by comparing a reception signal, which is output from the ultrasonic transducer element in the reference period, with the reference value.

A reference period is set upon detection of peeling. The reception signal of the ultrasonic transducer element and the reference value are compared with each other in the reference period. If the ultrasonic transducer element is not peeled off from the measurement target, the ultrasonic wave is reflected from the measurement target without passing through the air layer. Since the reference period is ended at an earlier time than the time of receiving a reflected wave from the measurement target, a reception signal corresponding to the reflected wave is not output from the ultrasonic transducer element in the reference period. Thus, it is detected that peeling has not occurred. When peeling occurs, the ultrasonic wave is reflected from the interface with the air before reaching the measurement target. A reception signal corresponding to the reflected wave is output from the ultrasonic transducer element in the reference period. The reception of a reflected wave is specified from the reception signal in the reference period. Accordingly, peeling is detected. Since peeling is determined in the reference period according to the comparison between the reception signal and the reference value, the processing time for determination is reduced. In addition, the difference between the air layer and the measurement target can be accurately detected from two conditions of the reflection time and the reflectance of the ultrasonic wave.

(2) The control device for an ultrasonic apparatus may further include an image processing section that is connected to an ultrasonic transducer element group including the ultrasonic transducer element and that generates an image signal based on a reception signal of the ultrasonic transducer element. Thus, the ultrasonic transducer element can be used not only in detecting peeling but also in forming an ultrasonic image. Ultrasonic transducer elements do not necessarily need to be disposed so as to be unique to the detection of peeling.

(3) In the control device for an ultrasonic apparatus, when the peeling is not detected by the peeling detecting section, the image processing section may generate an image signal based on a reception signal output from the ultrasonic transducer element in a period continuous to the reference period. If the ultrasonic transducer element is not peeled off, the ultrasonic wave is reflected from the measurement target. Since a reception signal corresponding to the reflected wave is not output from the ultrasonic transducer element in the reference period, peeling is not detected. The ultrasonic wave reflected from the measurement target is received by the ultrasonic transducer element in a period continuous to the reference period. The reception signal of the ultrasonic transducer element is used in forming an ultrasonic image. Thus, determination of peeling can be performed during the processing operation of image formation.

(4) When the peeling is detected by the peeling detecting section, the image processing section may hold an output of an image signal. When peeling is detected, the output of the image signal generated by the image processing section is held. Thus, when peeling is detected, a new ultrasonic image is not generated. Therefore, on a display device to display an ultrasonic image based on the image signal, it is possible to avoid the disturbance of display based on peeling.

(5) The control device for an ultrasonic apparatus may further include a selection section that selects the ultrasonic transducer element from the ultrasonic transducer element group. According to the selection, an ultrasonic transducer element used in the detection of peeling is specified. Therefore, it is possible to specify the position of an ultrasonic transducer element, which peels off, from the ultrasonic transducer element group. In this manner, the position of peeling can be accurately specified.

(6) The selection section may select an ultrasonic transducer element disposed on an outermost side in an arrangement of the ultrasonic transducer element group. When the ultrasonic transducer element group is in contact with the measurement target, it is thought that an ultrasonic transducer element disposed on the outermost side in the arrangement is likely to peel off most easily. Accordingly, if the ultrasonic transducer element disposed on the outermost side in the arrangement is selected, peeling is likely to be found. In this manner, it is possible to detect peeling efficiently.

(7) The selection section may select an ultrasonic transducer element disposed in a center in an arrangement of the ultrasonic transducer element group. When an ultrasonic transducer element located in the center of the arrangement is peeled off from the measurement target, it is thought that peeling occurs in a wide range including the center. Accordingly, since peeling is also expected in ultrasonic transducer elements disposed at other positions, peeling is assumed even if peeling detection processing is not performed. In this manner, subsequent processing operations can be omitted.

(8) The reference period setting section may define an end of the reference period at a time of receiving an ultrasonic wave reflected from an interface with the air. If the ultrasonic transducer element is peeled off from the measurement target, the ultrasonic wave is reflected from the interface with the air in the shortest path. If the ultrasonic transducer element is not peeled off from the measurement target, most ultrasonic waves are transmitted through the interface with the measurement target and are reflected from the inside of the measurement target. In the propagation time of the shortest path, the reflected wave of the ultrasonic wave is not received by the ultrasonic transducer element. Accordingly, if the end of the reference period is defined as a time of receiving the ultrasonic wave reflected from the interface with the air, a reception signal can be reliably identified according to whether or not there is peeling. In this manner, peeling can be reliably determined. In particular, since the interface with the air can be easily established, the reference period can be easily measured. Thus, it is possible to set the reference period accurately regardless of the individual difference of the measurement target.

(9) When comparing the reception signal with the reference value, the reception signal may be specified at a peak-to-peak value of a reception voltage. Thus, whether or not an ultrasonic wave has been received can be accurately detected in the reference period.

(10) The reference value setting section may set a peak-to-peak value of a voltage, which is output from the ultrasonic transducer element when receiving an ultrasonic wave reflected from the interface with the air, to the reference value, and the peeling detecting section may detect peeling if the peak-to-peak value of the reception voltage is equal to or greater than 0.2 times the reference value. If the ultrasonic transducer element is peeled off from the measurement target, the ultrasonic wave from the interface with the air is reflected toward the ultrasonic transducer element. On the other hand, if the ultrasonic transducer element is not peeled off from the measurement target, the ultrasonic wave from the interface with the measurement target is reflected toward the ultrasonic transducer element. While the acoustic impedance of the air and the acoustic impedance of the measurement target are greatly different, acoustic impedance matching is achieved between the measurement target and the contact body that defines the interface. Therefore, it is possible to accurately detect peeling by comparing the peak-to-peak values. In particular, since the interface with the air can be easily established, the reference value can be easily measured. Thus, it is possible to set the reference value accurately regardless of the individual difference of the measurement target.

(11) The reference value setting section may set a peak-to-peak value of a voltage, which is output from the ultrasonic transducer element when receiving an ultrasonic wave reflected from the interface with the measurement target, to the reference value, and the peeling detecting section may detect peeling if the peak-to-peak value of the reception voltage is equal to or greater than the reference value. If the ultrasonic transducer element is peeled off from the measurement target, the ultrasonic wave from the interface with the air is reflected toward the ultrasonic transducer element. On the other hand, if the ultrasonic transducer element is not peeled off from the measurement target, the ultrasonic wave from the interface with the measurement target is reflected toward the ultrasonic transducer element. While the acoustic impedance of the air and the acoustic impedance of the measurement target are greatly different, acoustic impedance matching is achieved between the measurement target and the contact body that defines the interface. Therefore, it is possible to accurately detect peeling by comparing the peak-to-peak values.

(12) The control device for an ultrasonic apparatus can be built into a probe. The probe may be connected to the control device for an ultrasonic apparatus and support the ultrasonic transducer element. The probe may include a display device that displays a position of peeling detected by the peeling detecting section. The user of the probe is notified of the position of peeling through the display device. The user can check the position of the peeling based on the display of the display device. Therefore, the user can eliminate the peeling accurately according to the display of the display device.

(13) The control device for an ultrasonic apparatus can be used by being built into an ultrasonic apparatus. In this case, the ultrasonic apparatus may include an apparatus body including the control device for an ultrasonic apparatus and a probe that is connected to the apparatus body and supports the ultrasonic transducer element.

(14) The ultrasonic apparatus may further include a display device that is connected to the apparatus body and displays a position of peeling detected by the peeling detecting section. The user of the ultrasonic apparatus is notified of the position of peeling through the display device. The user can check the position of the peeling based on the display of the display device. The user can eliminate the peeling accurately according to the display of the display device.

(15) Another aspect of the invention relates to a peeling detection method for an ultrasonic apparatus including: setting a reference value based on either a reflectance of an ultrasonic wave reflected from an interface with the air or a reflectance of an ultrasonic wave reflected from an interface with a measurement target; setting a reference period ended at an earlier time than a time of receiving an ultrasonic wave that is transmitted from an ultrasonic transducer element and is reflected from the measurement target; and detecting peeling of the ultrasonic transducer element by comparing a reception signal, which is output from the ultrasonic transducer element in the reference period, with the reference value.

A reference period is set upon detection of peeling. The reception signal of the ultrasonic transducer element and the reference value are compared with each other in the reference period. If the ultrasonic transducer element is not peeled off from the measurement target, the ultrasonic wave is reflected from the measurement target without passing through the air layer. Since the reference period is ended at an earlier time than the time of receiving a reflected wave, a reception signal corresponding to the reflected wave is not output from the ultrasonic transducer element in the reference period. It is detected that the ultrasonic transducer element has not been peeled off from the measurement target. When peeling occurs, the ultrasonic wave is reflected from the interface with the air before reaching the measurement target. Since a reception signal corresponding to the reflected wave is output from the ultrasonic transducer element, in the reference period, the reception of the reflected wave is specified in the reference period. Accordingly, peeling is detected. Since peeling is determined in the reference period according to the comparison between the reception signal and the reference value, the processing time for determination is reduced. In addition, the difference between the air layer and the measurement target can be accurately detected from two conditions of the reflection time and the reflectance of the ultrasonic wave.

BRIEF DESCRIPTION OF THE DRAWINGS

The invention will be described with reference to the accompanying drawings, wherein like numbers reference like elements.

Fig. 1 is a schematic diagram that schematically shows the configuration of an ultrasonic diagnostic apparatus.
Fig. 2 is a perspective view schematically showing the surface of an ultrasonic probe.
Fig. 3 is an enlarged partial plan view schematically showing the configuration of an ultrasonic device.
Fig. 4 is a sectional view taken along the line A-A of Fig. 3.
Fig. 5 is a block diagram schematically showing the circuit configuration of the ultrasonic diagnostic apparatus.
Fig. 6 is a block diagram schematically showing the configuration of a processing circuit.
Fig. 7 is a flowchart schematically showing the processing steps of the ultrasonic diagnostic process.
Fig. 8 is a flowchart schematically showing the processing steps of a start process.
Fig. 9 is a flowchart schematically showing the processing steps of an image update process.

Fig. 10 is a sequence diagram schematically showing the processing sequence of the ultrasonic diagnostic process.

Fig. 11 is a sequence diagram schematically showing the processing sequence of the ultrasonic diagnostic process continuous to Fig. 10.

Fig. 12 is a flowchart schematically showing a process for measuring a reference value and a reference period.

Fig. 13 is a waveform diagram showing the concept of start and end times of peeling detection.

DESCRIPTION OF EXEMPLARY EMBODIMENTS

Hereinafter, an embodiment of the invention will be described with reference to the accompanying diagrams. In addition, the present embodiment to be described below does not unduly limit the contents of the invention as defined in the appended claims, and all constituent elements described in the present embodiment are not necessarily indispensable as solving means of the invention.

(1) Overall configuration of an ultrasonic diagnostic apparatus

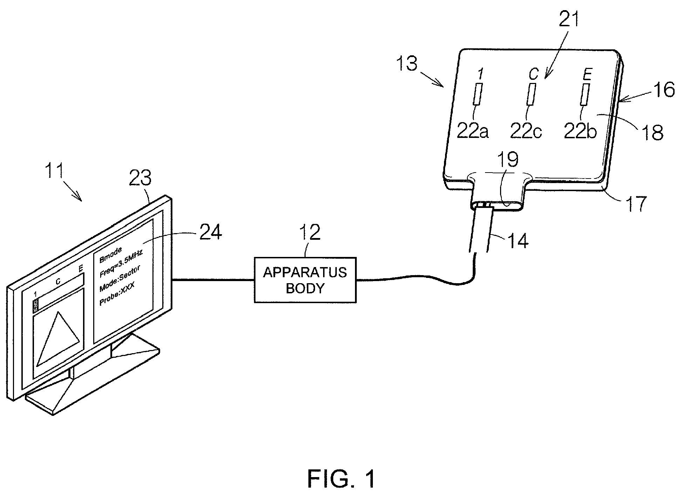

Fig. 1 schematically shows the configuration of a specific example of an electronic apparatus according to an embodiment of the invention, that is, the configuration of an ultrasonic diagnostic apparatus (ultrasonic apparatus) 11. The ultrasonic diagnostic apparatus 11 includes an apparatus body 12 and an ultrasonic probe (probe) 13. The apparatus body 12 and the ultrasonic probe 13 are connected to each other by a cable 14. The apparatus body 12 and the ultrasonic probe 13 perform transmission and reception of electrical signals therebetween through the cable 14.

The ultrasonic probe 13 includes a housing 16. The housing 16 includes a front side body 17 and a back side body 18. The front side body 17 and the back side body 18 are coupled to each other. Between the front side body 17 and the back side body 18, a cable port 19 is provided between the coupling surface of the front side body 17 and the coupling surface of the back side body 18. The cable 14 is disposed in the cable port 19. As will be described later, an ultrasonic device unit is supported by the housing 16.

A display unit (display device) 21 is provided in the housing 16 of the ultrasonic probe 13. The display unit 21 includes three lighting lamps 22a, 22b, and 22c. Each of the lighting lamps 22a, 22b, and 22c can be formed by an LED element. Each of the lighting lamps 22a, 22b, and 22c is turned on and off according to a control signal that is supplied.

The lighting lamps are arranged on one straight line in a determined direction. Here, the first and second lamps 22a and 22b are disposed at positions closest to the contour of the back side body 18 on one straight line, and the third lamp 22c is disposed at a central position that is equally distant from the first and second lamps 22a and 22b.

The display device 23 is connected to the apparatus body 12. A display panel 24 is provided in the display device 23. As will be described later, an image or a numerical value based on the ultrasonic wave detected by the ultrasonic probe 13 is displayed on the screen of the display panel 24. An imaging or digitizing detection result is displayed on the screen of the display panel 24.

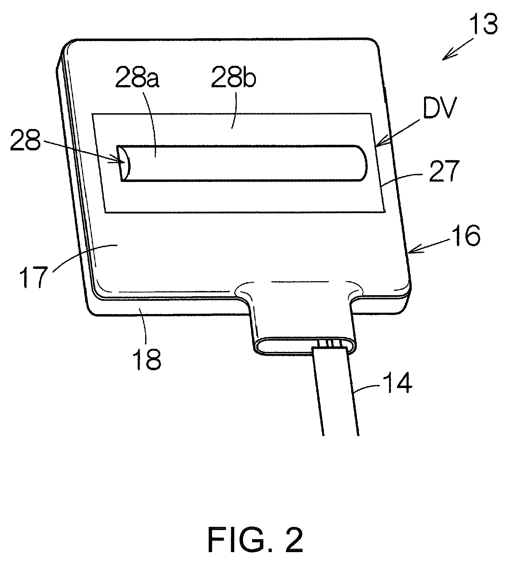

As shown in Fig. 2, an opening 26 is formed in the front side body 17 of the housing 16. The opening 26 faces the housing space provided in the housing 16. An ultrasonic device unit DV is disposed in the housing space. The ultrasonic device unit DV includes an ultrasonic device 27. The ultrasonic device 27 includes an acoustic lens 28. A partial cylindrical surface 28a is formed on the outer surface of the acoustic lens 28. The partial cylindrical surface 28a is surrounded by a flat plate portion 28b. The outer periphery of the flat plate portion 28b is attached to the housing 16 seamlessly all therearound. In this manner, the flat plate portion 28b functions as a part of the housing. The acoustic lens 28 is formed of, for example, silicone resin. The acoustic lens 28 has an acoustic impedance (for example, 1.0 [MRayl] to 1.5 [MRayl]) close to the acoustic impedance 1.5 [MRayl] of the body. The ultrasonic device 27 outputs an ultrasonic wave from the surface and receives a reflected wave of the ultrasonic wave.

(2) Configuration of the ultrasonic device

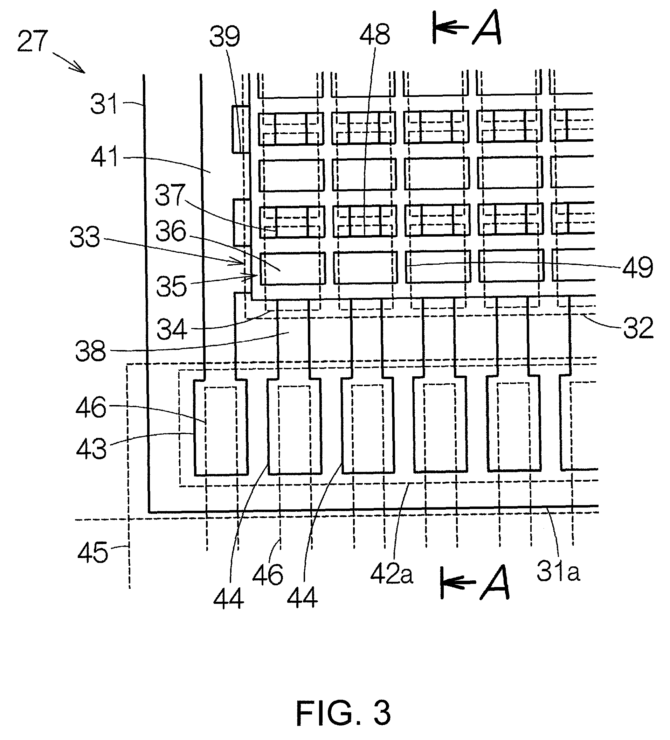

Fig. 3 schematically shows a plan view of the ultrasonic device 27 according to the embodiment. The ultrasonic device 27 includes a base 31. An element array 32 is formed on the base 31. The element array 32 is an array of ultrasonic transducer elements (hereinafter, referred to as "elements") 33. The array is formed in a matrix of a plurality of rows and columns. Here, only a part of the array is shown. In addition, in the array, a staggered arrangement may be established. In the staggered arrangement, a group of elements 33 in the even-numbered column may be shifted by 1/2 of the row pitch from a group of elements 33 in the odd-numbered column. The number of elements of one of the odd-numbered column and the even-numbered column may be one smaller than the number of elements of the other column.

Each element 33 includes a vibrating film 34. The details of the vibrating film 34 will be described later. In Fig. 3, the contour of the vibrating film 34 is drawn by a dotted line in plan view from a direction perpendicular to the film surface of the vibrating film 34 (in plan view from the thickness direction of the substrate). A piezoelectric element 35 is formed on the vibrating film 34. In the piezoelectric element 35, a piezoelectric film (not shown) is interposed between an upper electrode 36 and a lower electrode 37, as will be described later. These are superimposed in order. The ultrasonic device 27 is formed as one ultrasonic transducer element chip.

A plurality of first conductors 38 are formed on the surface of the base 31. The first conductors 38 extend in parallel in the column direction of the array. One first conductor 38 is assigned to the elements 33 in each column. One first conductor 38 is disposed in common to the elements 33 arranged in the column direction of the array. The first conductor 38 forms the lower electrode 37 for each element 33. For the first conductor 38, for example, a laminated film of titanium (Ti), iridium (Ir), platinum (Pt), and titanium (Ti) can be used. However, other conductive materials may be used for the first conductor 38.

A plurality of second conductors 39 are formed on the surface of the base 31. The second conductors 39 extend in parallel in the row direction of the array. One second conductor 39 is assigned to the elements 33 in each row. One second conductor 39 is connected in common to the elements 33 arranged in the row direction of the array. The second conductor 39 forms the upper electrode 36 for each element 33. Both ends of the second conductor 39 are connected to a pair of lead-out wiring lines 41, respectively. The lead-out wiring lines 41 extend in parallel in the column direction of the array. Accordingly, all of the second conductors 39 have the same length. In this manner, the upper electrode 36 is connected in common to the elements 33 in the entire matrix. The second conductor 39 can be formed of, for example, iridium (Ir). However, other conductive materials may be used for the second conductor 39.

The supply of power to the element 33 is switched for each column. A linear scan or a sector scan is realized according to such switching of power supply. Since the elements 33 in one column output ultrasonic waves at the same time, the number of elements in a column, that is, the number of rows of the array, can be determined according to the output level of the ultrasonic wave. For example, the number of rows may be set to about 10 to 15. In Fig. 3, three rows are drawn, and other rows are omitted. The number of columns of the array can be determined according to the spread of the scan range. For example, the number of columns may be set to 128 or 256. In Fig. 3, five columns are drawn, and other columns are omitted. The roles of the upper electrode 36 and the lower electrode 37 may be exchanged. That is, a lower electrode may be connected in common to the elements 33 in the entire matrix, and an upper electrode may be connected in common to respective columns of the array.

The contour of the base 31 has a first side 31a and a second side (not shown) that are a pair of parallel straight lines facing each other. A first terminal array 42a of one line is disposed between the first side 31a and the contour of the element array 32. A second terminal array 33b of one line is disposed between the second side 21b and the contour of the element array 32. By the first terminal array 42a, one line can be formed in parallel to the first side 31a. The first terminal array 42a is formed by a pair of upper electrode terminals 43 and a plurality of lower electrode terminals 44. The upper electrode terminals 43 are connected to the lead-out wiring lines 41. The lower electrode terminals 44 are connected to the second conductors 39. Similarly, a second terminal array (not shown) of one line may be disposed between the second side and the contour of the element array 32.

A flexible printed circuit board (hereinafter, referred to as a "circuit board") 45 is connected to the base 31. The circuit board 45 covers the first terminal array 42a. On one end of the circuit board 45, a conductive line, that is, a signal line 46 is formed corresponding to each upper electrode terminal 43 and each lower electrode terminal 44. The signal line 46 is separately bonded to each of the upper electrode terminal 43 and the lower electrode terminal 44 so as to face each of the upper electrode terminal 43 and the lower electrode terminal 44.

On the vibrating film 34, an electrode separation film 48 is disposed in parallel to the second conductors 39. The electrode separation film 48 extends in a band shape in the longitudinal direction of the second conductor 39. The electrode separation film 48 has an insulation property and a moisture-proof property. The electrode separation film 48 is formed of, for example, a moisture-proof insulating material, such as alumina ($Al_2O_3$) or silicon oxide ($SiO_2$). The electrode separation film 48 is formed so as to be separated on both sides of each second conductor 39 with the second conductor 39 interposed therebetween. Since the second conductor 39 crosses the first conductor 38 on the vibrating film 34, the electrode separation film 48 traverses the first conductor 38 on the vibrating film 34.

On the base 31, an insulating film 49 is formed outside the region of the vibrating film 34. The insulating film 49 extends in a band shape in the longitudinal direction of the first conductor 38. The insulating film 49 is disposed in parallel to the first conductors 38 in only a region outside the region of the vibrating film 34. The insulating film 49 is formed of, for example, a moisture-proof insulating material, such as alumina or silicon oxide. The insulating film 49 traverses the second conductor 39. The insulating film 49 is continuous to the electrode separation film 48.

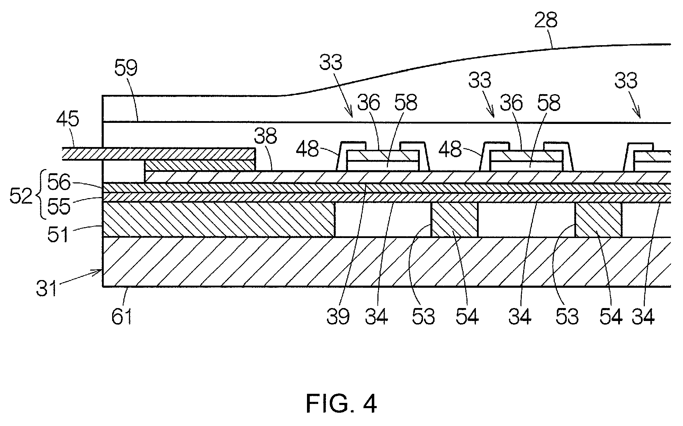

As shown in Fig. 4, the base 31 includes a substrate 51 and a flexible film 52. The flexible film 52 is formed on the entire surface of the substrate 51. On the substrate 51, an opening 53 is formed for each element 33. The openings 53 are disposed in the form of an array for the substrate 51. The contour of a region where the opening 53 is disposed corresponds to the contour of the element array 32. A partition wall 54 is provided between two adjacent openings 53. The adjacent openings 53 are partitioned by the partition wall 54.

The flexible film 52 is formed by a silicon oxide ($SiO_2$) layer 55 laminated on the surface of the substrate 51 and a zirconium oxide ($ZrO_2$) layer 56 laminated on the surface of the silicon oxide layer 55. The flexible film 52 is in contact with each opening 53. In this manner, a part of the flexible film 52 forms the vibrating film 34 corresponding to the contour of the opening 53. The thickness of the silicon oxide layer 55 can be determined based on the resonance frequency.

The first conductor 38, the piezoelectric film 58, and the second conductor 39 are laminated in order on the surface of the vibrating film 34. The piezoelectric film 58 can be formed of, for example, lead zirconate titanate (PZT). Other piezoelectric materials may be used for the piezoelectric film 58. Here, the piezoelectric film 58 covers the surface of the first conductor 38 completely under the second conductor 39. A short circuit between the first conductor 38 and the second conductor 39 can be avoided by the piezoelectric film 58. The surface of the piezoelectric film 58 is covered by the electrode separation film 48.

An acoustic matching layer 59 is laminated on the surface of the base 31. The acoustic matching layer 59 covers the element array 32. The thickness of the acoustic matching layer 59 is determined according to the resonance frequency of the vibrating film 34. For example, a silicone resin film can be used as the acoustic matching layer 59. The acoustic lens 28 is disposed on the acoustic matching layer 59. The acoustic lens 28 is in close contact with the surface of the acoustic matching layer 59 on the plane on the back side of the partial cylindrical surface 28a. The acoustic lens 28 is bonded to the base 31 by the acoustic matching layer 59. The generatrix of the partial cylindrical surface 28a is positioned in parallel to the first conductor 38. The curvature of the partial cylindrical surface 28a is determined according to the focal position of each ultrasonic wave transmitted from the elements 33 in one column that are connected to one second conductor 39.

A reinforcing plate 61 as a backing material is attached to the back surface of the base 31. The reinforcing plate 61 is formed in a flat plate shape. The back surface of the base 31 overlaps the surface of the reinforcing plate 61. The surface of the reinforcing plate 61 is bonded to the back surface of the base 31. At the time of such bonding, the reinforcing plate 61 may be bonded to the base 31 with an adhesive. The reinforcing plate 61 reinforces the rigidity of the base 31. The reinforcing plate 61 can include a rigid base, for example. Such a base may be formed of a metal material, such as a 42 alloy (iron-nickel alloy).

(3) Circuit configuration of the ultrasonic diagnostic apparatus

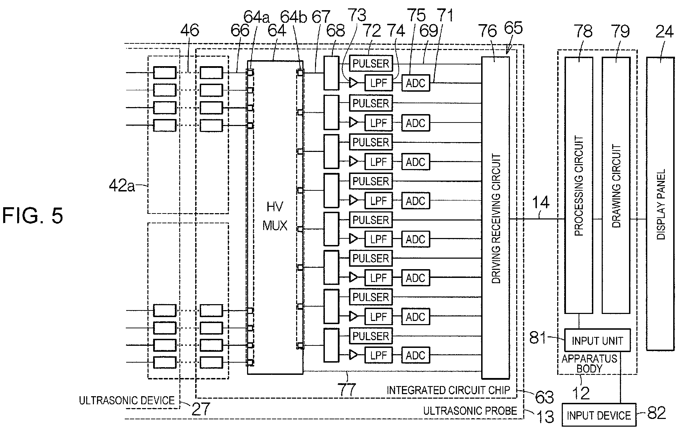

As shown in Fig. 5, the ultrasonic diagnostic apparatus 11 includes an integrated circuit chip 63 electrically connected to the ultrasonic device 27. The integrated circuit chip 63 includes a multiplexer 64 and a transmission and reception circuit 65. The multiplexer 64 includes a port group 64a on the ultrasonic device 27 side and a port group 64b on the transmission and reception circuit 65 side. A signal line 46 is connected to the port group 64a on the ultrasonic device 27 side through a wiring line 66. In this manner, the port group 64a is connected to the element array 32. Here, a predetermined number of signal lines 67 in the integrated circuit chip 63 are connected to the port group 64b on the transmission and reception circuit 65 side. The predetermined number corresponds to the number of columns of the elements 33 that are output at the same time in a scan. The multiplexer 64 manages the interconnection between a port on the cable 14 side and a port on the ultrasonic device 27 side.

The transmission and reception circuit 65 includes a predetermined number of selector switches 68. Each of the selector switch 68 is connected to the corresponding signal line 67. The transmission and reception circuit 65 includes a transmission path 69 and a reception path 71 for each selector switch 68. The transmission path 69 and the reception path 71 are connected in parallel to the selector switch 68. The selector switch 68 selectively connects the transmission path 69 or the reception path 71 to the multiplexer 64. A pulser 72 is provided in the transmission path 69. The pulser 72 outputs a pulse signal at a frequency corresponding to the resonance frequency of the vibrating film 34. An amplifier 73, a low pass filter (LPF) 74, and an analog-digital converter (ADC) 75 are provided in the reception path 71. The output signal of each element 33 is amplified and is converted into a digital signal.

The transmission and reception circuit 65 includes a driving receiving circuit 76. The transmission path 69 and the reception path 71 are connected to the driving receiving circuit 76. The driving receiving circuit 76 controls the pulser 72 according to the form of a scan. The driving receiving circuit 76 receives a digital signal of an output signal according to the form of a scan. The driving receiving circuit 76 is connected to the multiplexer 64 by a control line 77. The multiplexer 64 manages the interconnection based on the control signal supplied from the driving receiving circuit 76.

A processing circuit (control device for an ultrasonic apparatus) 78 is provided in the apparatus body 12. The processing circuit 78 can include a central processing unit (CPU) or a memory. The overall operation of the ultrasonic diagnostic apparatus 11 is controlled according to the processing of the processing circuit 78. The processing circuit 78 controls the driving receiving circuit 76 according to the instruction input from the user. The processing circuit 78 generates an image according to the output signal of the element 33. An image is specified by the drawing data.

A drawing circuit 79 is provided in the apparatus body 12. The drawing circuit 79 is connected to the processing circuit 78. The display panel 24 is connected to the drawing circuit 79. The drawing circuit 79 generates a driving signal according to the drawing data generated by the processing circuit 78. The driving signal is transmitted to the display panel 24. As a result, an image is projected onto the display panel 24.

An input unit 81 is provided in the apparatus body 12. The input unit 81 is connected to the processing circuit 78. An input device 82, such as a keyboard, a mouse, or a touch-screen panel, may be connected to the input unit 81. A command or data from the input device can be input to the processing circuit 78. The processing circuit 78 can operate based on the input command or data.

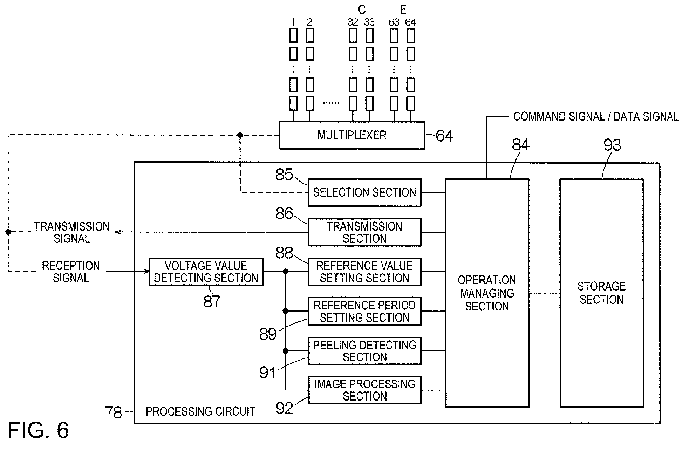

As shown in Fig. 6, the processing circuit 78 includes an operation managing section 84. The input unit 81 is connected to the operation managing section 84. The operation managing section 84 performs overall control of the processing circuit 78. The operation managing section 84 performs arithmetic processing based on the command signal or data signal input from the input unit 81.

The processing circuit 78 includes a selection section 85. The selection section 85 is connected to the operation managing section 84. The multiplexer 64 is connected to the selection section 85 through the driving receiving circuit 76. The selection section 85 selects one or more elements 33 from elements belonging to the element array 32. A selection signal is supplied to the multiplexer 64 from the selection section 85 when selecting the element 33. The selection section 85 generates a selection signal according to the instruction of the operation managing section 84.

The processing circuit 78 includes a transmission section 86. The transmission section 86 is connected to the operation managing section 84. The transmission section 86 supplies a transmission signal toward the multiplexer 64. Each element 33 transmits an ultrasonic wave in response to the supply of the transmission signal. The transmission section 86 generates a transmission signal according to the instruction of the operation managing section 84.

The processing circuit 78 includes a voltage value detecting section 87. The multiplexer 64 is connected to the voltage value detecting section 87 through the driving receiving circuit 76. The voltage value detecting section 87 receives the reception signal supplied from each element 33. According to the supplied reception signal, a voltage value for each time is specified. The time can be determined according to the resolution of the ADC 75, for example. The resolution of ADC 75 is determined according to the sampling frequency.

The processing circuit 78 includes a reference value setting section 88. The reference value setting section 88 is connected to the voltage value detecting section 87. The reference value setting section 88 sets a reference value according to the output of the voltage value detecting section 87. When setting the reference value, the reference value setting section 88 specifies a peak-to-peak value of the voltage output from the element 33. The reception of the ultrasonic wave is specified at the maximum value of the peak-to-peak value.

The operation managing section 84 is connected to the reference value setting section 88. The operation managing section 84 controls the operation of the reference value setting section 88. As will be described later, the peak-to-peak value of the voltage that is output from the element 33 when receiving the ultrasonic wave reflected from the interface with the air is specified according to the control of the operation managing section 84. In this manner, the reference value reflects the reflectance of the ultrasonic wave reflected from the interface with the air.

The processing circuit 78 includes a reference period setting section 89. The reference period setting section 89 is connected to the voltage value detecting section 87. The reference period setting section 89 sets the reference period according to the output of the voltage value detecting section 87. When setting the reference period, the reference period setting section 89 specifies a time of receiving the ultrasonic wave after transmission of the ultrasonic wave. When specifying the time, the reference period setting section 89 specifies the maximum value of the peak-to-peak value of the voltage output from the element 33. The operation managing section 84 is connected to the reference period setting section 89. The operation managing section 84 controls the operation of the reference period setting section 89. As will be described later, according to the control of the operation managing section 84, the end of the reference period is defined at the time of receiving the ultrasonic wave reflected from the interface with the air. When the end of the reference period is defined as described above, the reference period is ended at an earlier time than the time of receiving the ultrasonic wave that is transmitted from the element 33 and is reflected from the measurement target.

The processing circuit 78 includes a peeling detecting section 91. The peeling detecting section 91 is connected to the voltage value detecting section 87. The peeling detecting section 91 detects the peeling of the element 33 by comparing the reference value with the reception signal output from the element 33 in the reference period. When comparing the reception signal with the reference value, the reception signal is specified at the peak-to-peak value of the reception voltage. The peeling detecting section 91 detects peeling if the peak-to-peak value of the reception voltage is equal to or greater than 0.2 times the reference value.

The processing circuit 78 includes an image processing section 92. The image processing section 92 is connected to the voltage value detecting section 87. The image processing section 92 generates image data according to the output of the voltage value detecting section 87. The operation managing section 84 is connected to the image processing section 92. The operation managing section 84 controls the operation of the image processing section 92. According to the control of the operation managing section 84, the image processing section 92 generates image data based on the reception signal output from the element 33 in a period that is continuous to the reference period. When the peeling is detected by the peeling detecting section 91, the image processing section 92 holds the output of image data.

The processing circuit 78 includes a storage section 93. The storage section 93 is connected to the operation managing section 84. A reference value and a reference period are stored in the storage section 93.

(4) Operation of the ultrasonic diagnostic apparatus

The operation of the ultrasonic diagnostic apparatus 11 will be described briefly. The ultrasonic probe 13 is pressed against a measurement target before the ultrasonic diagnostic process is started. Here, for example, a body is assumed to be the measurement target. The acoustic lens 28 is in close contact with the skin of the body. Between the skin and the acoustic lens 28 that have been in close contact with each other, water or other acoustic coupling materials may be disposed. Since the acoustic impedance of the acoustic lens 28 is 1.0 [MRayl] to 1.5 [MRayl], the acoustic impedance of the acoustic lens 28 matches the acoustic impedance 1.5 [MRayl] of the body. Accordingly, each element 33 is acoustically coupled to the body. As long as the acoustic lens 28 is in close contact with the skin, the reflection of the ultrasonic wave is maximally suppressed on the interface of the acoustic lens 28. In this manner, an ultrasonic wave can reach an organ in the body.

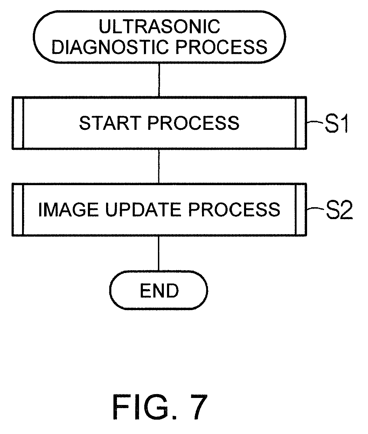

As shown in Fig. 7, the ultrasonic diagnostic process is started, for example, in response to the operation of the input device 82. When the ultrasonic diagnostic process is started, a start process is performed in step S1. In the start process, "peeling" of the element array 32 is detected. The details of detection of "peeling" will be described later. Subsequent to the start process, an image update process is performed in step S2. In the image update process, an image is formed for each video frame.

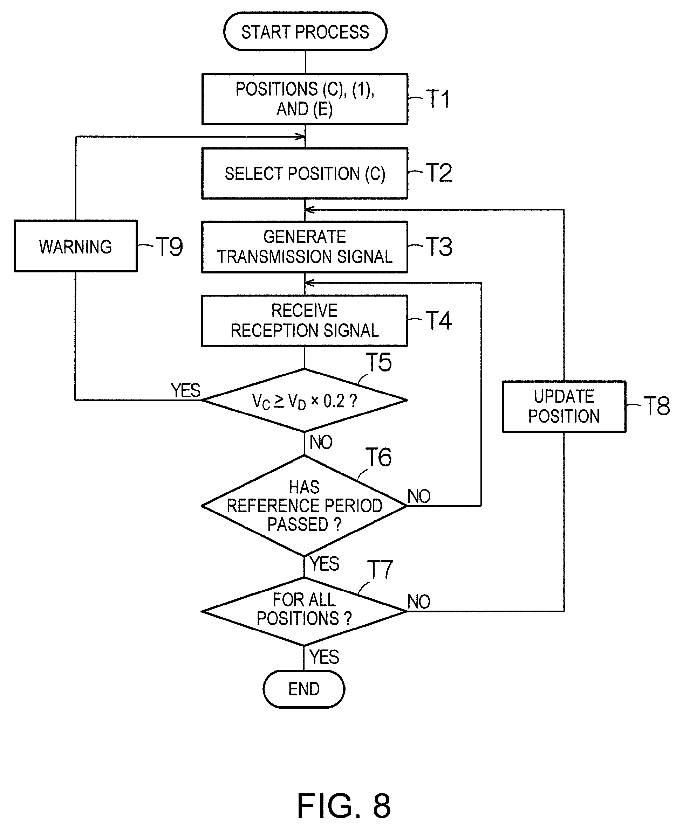

As shown in Fig. 8, in the start process, the operation managing section 84 specifies the detection position of "peeling" in step T1. Here, for example, as shown in Fig. 6, the element array 32 includes 64 element columns in the scanning direction. A position (C) corresponds to one element column (thirty-second column) disposed in the center in the scanning direction in the element array 32. Positions (1) and (E) correspond to one element column (first column) and one element column (sixty-fourth column), respectively, which are disposed on the outermost side in the scanning direction in the element array 32.

In step T2, the selection section 85 selects, for example, the position (C) according to the control of the operation managing section 84. In step T3, the transmission section 86 generates a transmission signal according to the control of the operation managing section 84. The transmission signal is supplied to the element column placed at the position (C). According to the transmission signal, ultrasonic waves are transmitted from the element column placed at the position (C). In the element column, a column of elements 33 for only transmission and a column of elements 33 for only reception may be formed. In step T4, the voltage value detecting section 87 receives a reception signal. A voltage value $V_C$ of the reception signal is specified. Then, in step T5, the peeling detecting section 91 compares the voltage value $V_C$ of the reception signal with a reference value $V_D$. If the voltage value $V_C$ of the reception signal is equal to or greater than 0.2 times the reference value $V_D$, the peeling detecting section 91 detects peeling. If the voltage value $V_C$ of the reception signal is less than 0.2 times the reference value $V_D$, peeling is denied. Until the reference period passes in step T6, peeling is determined for each voltage value $V_C$. When the reference period has passed without peeling being detected, the processing operation proceeds to step T7. Since the positions (1) and (E) remain, a position is updated in step T8, and the selection section 85 selects the position (1) according to the control of the operation managing section 84. When the reference period has passed without peeling being detected at the position (C), the position (1), and the position (E) according to such a series of processing operation, the start process is ended.

When peeling is detected in step T5 before the reference period passes in step T6, the peeling detecting section 91 generates a peeling warning signal in step T9. The peeling warning signal is supplied to, for example, the display unit 21. For example, when peeling is detected at the position (C), the display unit 21 turns on the third lamp 22c corresponding to the position (C). When peeling is detected at the position (1), the display unit 21 turns on the first lamp 22a corresponding to the position (1). When peeling is detected at the position (E), the display unit 21 turns on the second lamp 22b corresponding to the position (E).

The operator of the ultrasonic diagnostic apparatus 11 can know the "peeling" of the element array 32 according to the lighting of the third lamp 22c, the first lamp 22a, or the second lamp 22b. "Peeling" of the element array 32 forms an air layer between the acoustic lens 28 and the skin to eliminate close contact. Due to "peeling", ultrasonic waves are strongly reflected from the interface with the air. Accordingly, since the ultrasonic waves cannot reach an organ in the body, an unnecessary shade is formed in the ultrasonic image. The operator who viewed the warning display can attempt a close contact between the acoustic lens 28 and the skin of the body. When peeling is detected, the selection section 85 selects the position (C) again in step T2. It is determined again whether or not there is peeling at the position (C), the position (1), and the position (E).

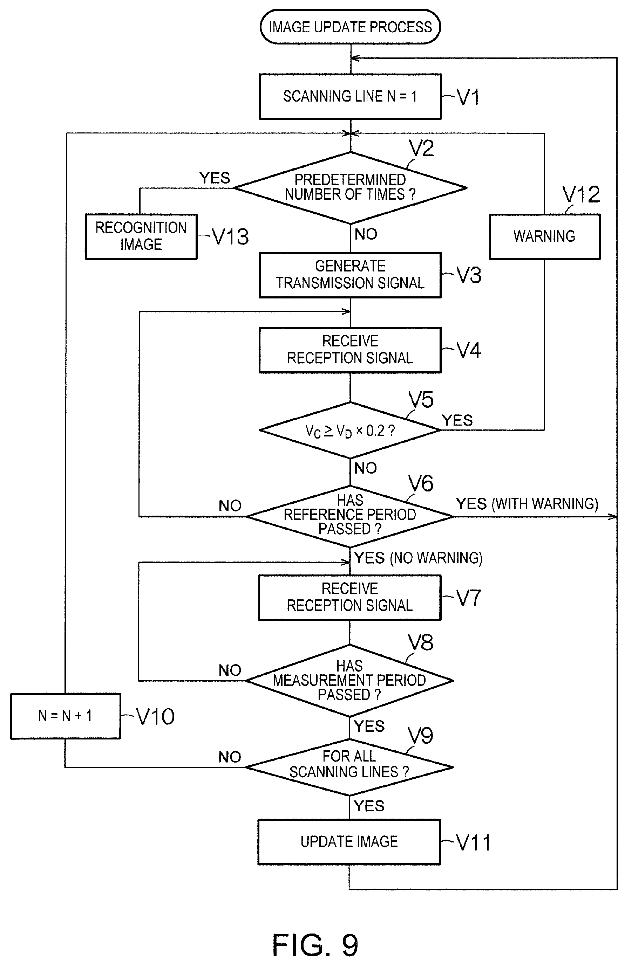

As shown in Fig. 9, the operation managing section 84 sets a first scanning line (N = 1) in step V1 in the image update process. Here, 64 element columns are formed in the scanning direction, and 61 scanning lines per video frame are formed. Here, the number of element columns or the number of scanning lines can be appropriately set according to the specification of the ultrasonic diagnostic apparatus 11 or the required image quality or resolution. In step V2, the operation managing section 84 determines the number of times of the output of the warning signal. Since a tendency signal has not yet been output in the first scanning line, the processing operation proceeds to step V3. In step V3, the transmission section 86 generates a transmission signal according to the control of the operation managing section 84. The transmission signal is supplied to, for example, the element column placed at the position (1). According to the transmission signal, ultrasonic waves are transmitted from the element column placed at the position (1). In step V4, the voltage value detecting section 87 receives a reception signal. The voltage value $V_C$ of the reception signal is specified. Then, in step V5, the peeling detecting section 91 compares the voltage value $V_C$ of the reception signal with the reference value $V_D$. If the voltage value $V_C$ of the reception signal is equal to or greater than 0.2 times the reference value $V_D$, the peeling detecting section 91 detects peeling. If the voltage value $V_C$ of the reception signal is less than 0.2 times the reference value $V_D$, peeling is denied. Until the reference period passes in step V6, peeling is determined for each voltage value $V_C$. When the reference period has passed without peeling being detected, the processing operation proceeds to step V7. In step V7, the voltage value detecting section 87 receives a reception signal. In step V8, the voltage value $V_C$ of the reception signal is specified until a measurement period continuous to the reference period passes. The image processing section 92 generates image data according to the maximum value of the voltage value $V_C$. When the measurement period has passed, image data is stored in the storage section 93.

In step V9, the operation managing section 84 determines whether or not the voltage value $V_C$ is stored for all scanning lines (N = 1, 2, ..., 61). In step V10, the next scanning line (N = 2) is selected. The processing operation of steps V2 to V9 is repeated. When the voltage value $V_C$ of 61 scanning lines is stored in step V9, an image of one video frame is formed according to the voltage value $V_C$. In step V11, image data is supplied to the drawing circuit 79. An image is updated on the screen of the display panel 24. Then, the process is repeated from step V1. The next video frame is formed. In this manner, the formation of a video frame is repeated. As a result, an image is formed.

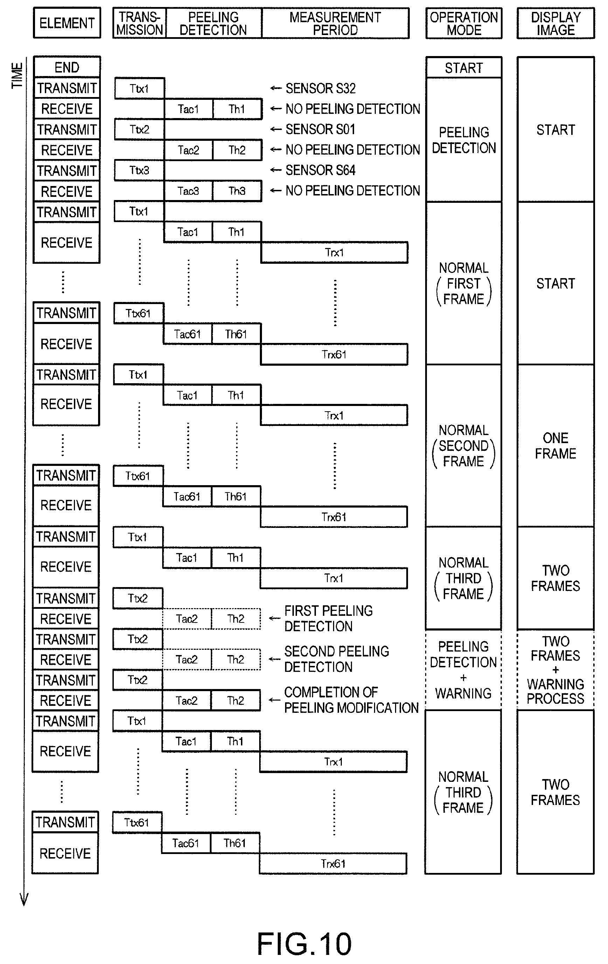

When peeling is detected in step V5 before the reference period passes in step V6, the peeling detecting section 91 generates a peeling warning signal in step V12. The peeling warning signal is supplied to, for example, the display unit 21. The display unit 21 turns on the first lamp 22a, the second lamp 22b, and the third lamp 22c. One of the first lamp 22a, the second lamp 22b, and the third lamp 22c may be selected according to the position of the element column. In addition, as shown in Fig. 10, the operation managing section 84 may generate a warning image signal. The warning image signal is supplied to the drawing circuit 79. A warning image is added to the screen of the display panel 24. The operator who viewed the warning display or the warning image can attempt the establishment of a close contact between the acoustic lens 28 and the skin of the body. In this case, the output of the image signal generated by the image processing section 92 is held. Thus, when peeling is detected, a new ultrasonic image is not generated. Accordingly, on the screen of the display panel 24 , it is possible to avoid the disturbance of the display based on peeling. In step V6, when the operation managing section 84 recognizes the elimination of peeling in the corresponding scanning line in the reference period, the processing operation returns to step V1. Then, the formation of one video frame is performed again from the scanning line (N = 1).

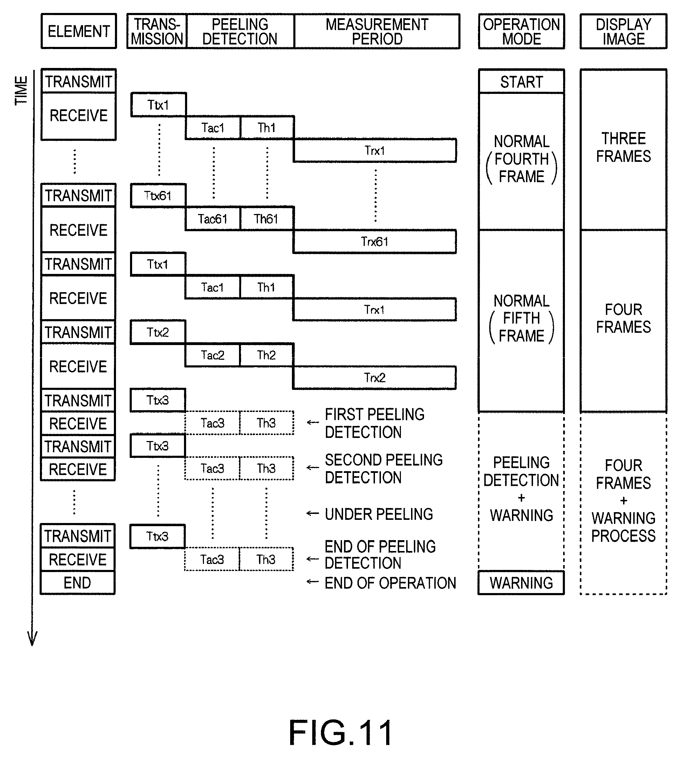

When the number of times of the output of the warning signal reaches a predetermined number in step V2 , the operation managing section 84 may generate an image signal of "peeling unresolved" in step V13. The image signal is supplied to the drawing circuit 79. On the screen of the display panel 24, as shown in Fig. 11, a recognition image of "peeling unresolved" is added without updating the image of the video frame. The recognition image may prompt the re-start of the ultrasonic diagnostic process. The operation of the ultrasonic diagnostic process is ended while maintaining the image of the video frame and the recognition image in this manner.

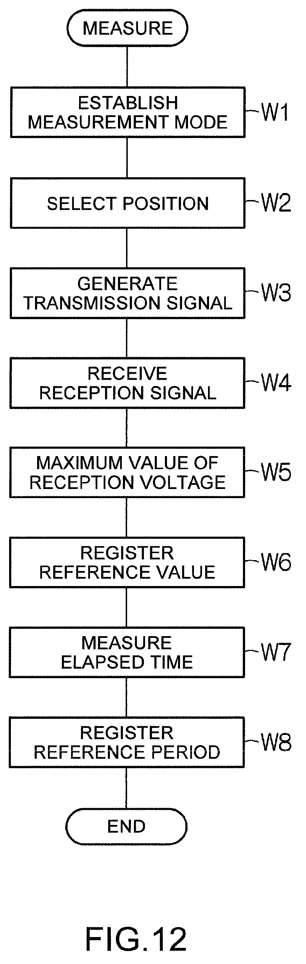

As shown in Fig. 12, a reference value and a reference period may be set based on measurement. For example, such measurement may be performed prior to shipment of products in the production site. Before the measurement, an interface with the air is established in the acoustic lens 28. At the time of establishment, the measurer may hold up the ultrasonic probe 13 in the space, for example. The measurement process is started in response to the operation of the input device 82. In step W1, a measurement mode is established in the operation managing section 84.

In step W2, the operation managing section 84 specifies a detection position in the measurement. Here, the selection section 85 selects, for example, the position (C), according to the control of the operation managing section 84. Since each element column has the same ultrasonic characteristics as the acoustic lens 28, the reference value and the reference period that are measured in one element column can be used in common to all element columns. In step W3, the transmission section 86 generates a transmission signal according to the control of the operation managing section 84. The transmission signal is supplied to the element column placed at the position (C). The wave number of the transmitted ultrasonic wave is set to 0.5 or more.

In step W4, the voltage value detecting section 87 receives a reception signal. The voltage value $V_C$ of the received reception signal is specified. Since the ultrasonic wave is maximally reflected from the interface with the air, the voltage value $V_C$ of the reception signal indicates a maximum value at the time of reception of ultrasonic waves, and decreases therefrom. In step W5, the reference value setting section 88 specifies the maximum value of the peak-to-peak value of the reception voltage. The reference value setting section 88 sets the maximum value as the reference value. In step W6, the operation managing section 84 registers the reference value set by the reference value setting section 88 in the storage section 93.

In step W7, the reference period setting section 89 measures an elapsed time from the output of the transmission signal to the maximum value of the peak-to-peak value of the reception voltage. The reference period setting section 89 sets the elapsed time as the reference period. In step W8, the operation managing section 84 registers the reference period set by the reference period setting section 89 in the storage section 93.

Here, the peak-to-peak value of the voltage output from the element 33 when receiving the ultrasonic wave reflects the reflectance of the ultrasonic wave. The reflectance of the ultrasonic wave is calculated based on the acoustic impedance of a first medium, through which the ultrasonic wave propagates, and the acoustic impedance of a second medium, which is in contact with the interface of the first medium. For example, when the acoustic lens 28 has an acoustic impedance of 1.0 [MRayl], the ultrasonic wave propagating through the acoustic lens 28 is reflected from the interface with the air with a reflectance $\gamma$ according to the following equation since the acoustic impedance of the air is $4.28 \times 10^4$ [MRayl].

$$\gamma = \frac{Z_{AIR} - Z_{LENS}}{Z_{AIR} + Z_{LENS}} = -0.9994$$

When this is set as the reference value $V_D$, the ultrasonic wave propagating through the acoustic lens 28 is reflected from the interface with the body with a reflectance $\gamma$ according to the following equation.

$$\gamma = \frac{Z_{BIO} - Z_{LENS}}{Z_{BIO} + Z_{LENS}} = 0.2$$

Since these reflectances are reflected on the voltage value $V_C$ of the reception signal, it is possible to detect peeling if the peak-to-peak value of the reception voltage is equal to or greater than 0.2 times the reference value. For example, when the acoustic lens 28 has an acoustic impedance of 1.5 [MRayl], the ultrasonic wave propagating through the acoustic lens 28 is reflected with a reflectance $\gamma$ according to the following equation on the interface with the body.

$$\gamma = \frac{Z_{BIO} - Z_{LENS}}{Z_{BIO} + Z_{LENS}} = 0$$

Therefore, in the same manner as described above, it is possible to detect peeling if the peak-to-peak value of the reception voltage is equal to or greater than 0.2 times the reference value.

Here, a reference period $T_{AC}$ [second] may be defined according to the following equation.

$$T_{AC} = \left( \frac{Dm}{Cm} + \frac{D_{lens}}{C_{lens}} \right) \times 2$$

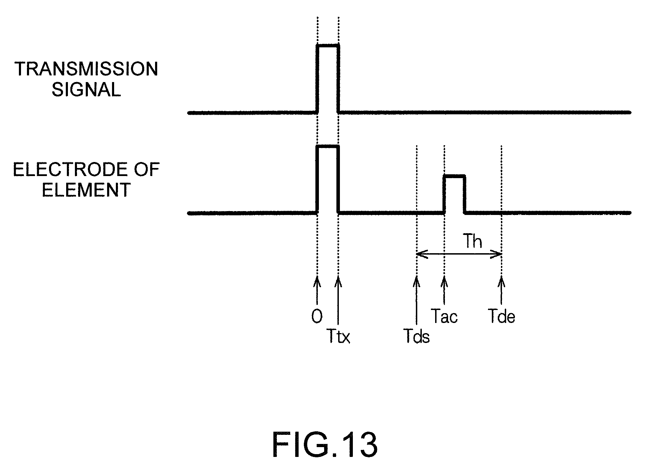

In addition, Dm indicates the thickness [m] of the acoustic matching layer 59, and Cm indicates the sound speed [m/s] of the acoustic matching layer 59. Similarly, $D_{lens}$ indicates the thickness [m] of the acoustic lens 28, and $C_{lens}$ indicates the sound speed [m/s] of the acoustic lens 28. As shown in Fig. 13, a start time Tds and an end time Tde of peeling detection may be set according to the tolerance $\alpha$ of the ultrasonic probe 13.

$$Tds = T_{AC} \times (1 - \alpha)$$

$$Tde = T_{AC} \times (1 + \alpha) + Ttx$$

$$Ttx = \frac{1}{ft} \times n$$

In this case, Ttx indicates a transmission period [second], ft indicates a transmission frequency [Hz], and n indicates a wave number [wave].

In the ultrasonic diagnostic apparatus 11, a reference period is set upon detection of peeling. The reception signal of the element 33 and the reference value are compared with each other in the reference period. If the acoustic lens 28 is in close contact with the body, most ultrasonic waves are reflected from the organ in the body. Since the reference period is ended at an earlier time than the time of receiving a reflected wave from the organ, a reception signal corresponding to the reflected wave is not output from the element 33 in the reference period. Accordingly, peeling is denied. When peeling occurs, the ultrasonic wave is reflected from the interface with the air before reaching the organ in the body. Since the reception signal corresponding to the reflected wave is output from the element 33 in the reference period, a voltage value corresponding to the reflected wave is detected in the reference period. Accordingly, peeling is detected. Since peeling is determined in the reference period according to the comparison between the reception signal and the reference value, the processing time for determination is reduced. The difference between the air layer and the body can be accurately detected from two conditions of the reflection time and the reflectance of the ultrasonic wave.

As described above, the peeling detecting section 91 detects peeling based on the voltage value detected by the voltage value detecting section 87. Similarly, the image processing section 92 generates image data based on the voltage value detected by the voltage value detecting section 87. Thus, the element 33 can be used not only in detecting peeling but also in forming an ultrasonic image. Ultrasonic transducer elements do not necessarily need to be disposed so as to be unique to the detection of peeling. In this case, the size of the ultrasonic device 27 is reduced. Accordingly, the wiring structure is simplified. In addition, peeling can be determined during the processing operation for image formation.

As described above, in the start process, the selection section 85 selects a specific element 33. It is determined whether or not there is peeling at the position (C), the position (1), and the position (E). The position of peeling can be accurately specified. The selection section 85 selects element columns at the position (1) and the position (E) that are disposed on the outermost side in the arrangement of the element array 32. When the acoustic lens 28 is in contact with the body, it is thought that the element 33 disposed on the outermost side in the arrangement is likely to peel off most easily. Accordingly, if the elements 33 placed at the positions (1) and (E) are selected, peeling is easily found. In this manner, it is possible to detect peeling efficiently. In addition, when the element 33 at the position (C) located in the center of the arrangement is peeled off from the body, it is thought that peeling occurs in a wide range including the center. Since peeling is also expected in the element 33 placed at the position (1) or the position (E), peeling is assumed even if peeling detection processing is not performed at other positions. In this manner, subsequent processing operations can be omitted.

If the acoustic lens 28 is peeled off from the body, the ultrasonic wave is reflected from the interface with the air. The ultrasonic wave is reflected in the shortest path. If the acoustic lens 28 is not peeled off from the body, most ultrasonic waves are transmitted through the interface with the body and are reflected from the organ inside the body. In the propagation time of the shortest path, the reflected wave of the ultrasonic wave is not received by the element 33. Accordingly, if the end of the reference period is defined as a time of receiving the ultrasonic wave reflected from the interface with the air, a reception signal can be reliably identified according to whether or not there is peeling. In this manner, peeling can be reliably determined. In addition, as described above, since the interface with the air can be easily established, the reference period can be easily measured. Thus, it is possible to set the reference period accurately regardless of the individual difference of the body.

When comparing the reception signal with the reference value, the reception signal is specified at the peak-to-peak value of the reception voltage. Thus, whether or not an ultrasonic wave has been received can be accurately detected in the reference period. If the acoustic lens 28 is peeled off from the body, the ultrasonic wave from the interface with the air is reflected toward the element 33. On the other hand, if the acoustic lens 28 is in close contact with the body, the ultrasonic wave from the interface with the body is reflected toward the element 33. While the acoustic impedance of the air and the acoustic impedance of the organ are greatly different, acoustic impedance matching is achieved between the body and the acoustic lens 28 that defines the interface. Therefore, it is possible to accurately detect peeling by comparing the peak-to-peak values. In particular, since the interface with the air can be easily established, the reference value can be easily measured. Thus, it is possible to set the reference value accurately regardless of the individual difference of the body.

In the ultrasonic probe 13, the first lamp 22a, the second lamp 22b, and the third lamp 22c are respectively disposed corresponding to the positions (1), (E), and (C) in the element column. When the housing 16 is pressed toward the body at the position of the first lamp 22a, contact between the acoustic lens 28 and the body is strengthened at a position corresponding to the element column of the position (1). When the housing 16 is pressed toward the body at the position of the second lamp 22b, contact between the acoustic lens 28 and the body is strengthened at a position corresponding to the element column of the position (E). When the housing 16 is pressed toward the body at the position of the third lamp 22c, contact between the acoustic lens 28 and the body is strengthened at a position corresponding to the element column of the position (C). Therefore, the user can eliminate peeling accurately according to the display of the display unit 21.

In the reference value setting section 88, the peak-to-peak value of the voltage that is output from the element 33 when receiving the ultrasonic wave reflected from the interface with the body may be specified as a reference value. In this case, the reference value reflects the reflectance of the ultrasonic wave reflected from the interface with the body. When the peak-to-peak value of the voltage that is output from the element 33 when receiving the ultrasonic wave reflected from the interface with the body is set to the reference value as described above, the peeling detecting section detects peeling if the peak-to-peak value of the reception voltage is equal to or greater than the reference value.

While the present embodiment has been described in detail as described above, it could be easily understood by those skilled in the art that various changes and modifications thereof could be made without departing from novel matters and effects of the invention. Accordingly, all of such modification examples still are included in the range of the invention. For example, in the specification or diagrams, a term which is described at least once together with different terms having a broader meaning or the same meaning can be replaced with the different terms in any parts of the specification or diagrams. The configurations and operations of the ultrasonic device unit DV, the ultrasonic device 27, the element 33, and the like are not limited to those described in the present embodiment, and various modifications can be made. The processing circuit 78 may be formed using a central processing unit (CPU). In this case, each functional block in the CPU may be realized by arithmetic processing of software.

**Claims**

1. A control device for an ultrasonic apparatus, comprising:

   a reference value setting section that sets a reference value based on either a reflectance of an ultrasonic wave reflected from an interface with the air or a reflectance of an ultrasonic wave reflected from an interface with a measurement target;
   a reference period setting section that sets a reference period ended at an earlier time than a time of receiving an ultrasonic wave that is transmitted from an ultrasonic transducer element and is reflected from the measurement target; and
   a peeling detecting section that detects peeling of the ultrasonic transducer element by comparing a reception signal, which is output from the ultrasonic transducer element in the reference period, with the reference value.

2. The control device for an ultrasonic apparatus according to claim 1, further comprising:

   an image processing section that is connected to an ultrasonic transducer element group including the ultrasonic transducer element and that generates an image signal based on a reception signal of the ultrasonic transducer element.

3. The control device for an ultrasonic apparatus according to claim 2,
   wherein, when the peeling is not detected by the peeling detecting section, the image processing section generates an image signal based on a reception signal output from the ultrasonic transducer element in a period continuous to the reference period.

4. The control device for an ultrasonic apparatus according to claim 2 or 3,
   wherein, when the peeling is detected by the peeling detecting section, the image processing section holds an output of an image signal.

5. The control device for an ultrasonic apparatus according to any one of the preceding claims, further comprising:

   a selection section that selects the ultrasonic transducer element from the ultrasonic transducer element group.

6. The control device for an ultrasonic apparatus according to claim 5,
   wherein the selection section selects an ultrasonic transducer element disposed on an outermost side in an arrangement of the ultrasonic transducer element group.

7. The control device for an ultrasonic apparatus according to claim 5 or 6,
   wherein the selection section selects an ultrasonic transducer element disposed in a center in an arrangement of the ultrasonic transducer element group.

8. The control device for an ultrasonic apparatus according to any one of the preceding claims,
   wherein the reference period setting section defines an end of the reference period at a time of receiving an ultrasonic wave reflected from an interface with the air.

9. The control device for an ultrasonic apparatus according to any one of the preceding claims,
   wherein, when comparing the reception signal with the reference value, the reception signal is specified at a peak-to-peak value of a reception voltage.

10. The control device for an ultrasonic apparatus according to any one of the preceding claims,
    wherein the reference value setting section sets a peak-to-peak value of a voltage, which is output from the ultrasonic transducer element when receiving an ultrasonic wave reflected from the interface with the air, to the reference value, and
    the peeling detecting section detects peeling if the peak-to-peak value of the reception voltage is equal to or greater than 0.2 times the reference value.

11. The control device for an ultrasonic apparatus according to any one of the preceding claims,
    wherein the reference value setting section sets a peak-to-peak value of a voltage, which is output from the ultrasonic transducer element when receiving an ultrasonic wave reflected from the interface with the measurement target, to the reference value, and the peeling detecting section detects peeling if the peak-to-peak value of the reception voltage is equal to or greater than the reference value.

12. A probe that is connected to the control device for an ultrasonic apparatus according to any one of the preceding claims and supports the ultrasonic transducer element, the probe comprising:

a display device that displays a position of peeling detected by the peeling detecting section.

13. An ultrasonic apparatus, comprising:

an apparatus body including the control device for an ultrasonic apparatus according to any one of claims 1 to 11; and
a probe that is connected to the apparatus body and supports the ultrasonic transducer element.

14. The ultrasonic apparatus according to claim 13 , further comprising:

a display device that is connected to the apparatus body and displays a position of peeling detected by the peeling detecting section.

15. A peeling detection method for an ultrasonic apparatus, comprising:

setting a reference value based on either a reflectance of an ultrasonic wave reflected from an interface with the air or a reflectance of an ultrasonic wave reflected from an interface with a measurement target;
setting a reference period ended at an earlier time than a time of receiving an ultrasonic wave that is transmitted from an ultrasonic transducer element and is reflected from the measurement target; and
detecting peeling of the ultrasonic transducer element by comparing a reception signal, which is output from the ultrasonic transducer element in the reference period, with the reference value.

FIG. 1

FIG. 2

FIG. 3

FIG. 4

FIG. 5

FIG. 6

ULTRASONIC DIAGNOSTIC PROCESS

START PROCESS ─S1

IMAGE UPDATE PROCESS ─S2

END

# FIG. 7

START PROCESS

POSITIONS (C), (1), AND (E) — T1

SELECT POSITION (C) — T2

WARNING — T9

GENERATE TRANSMISSION SIGNAL — T3

RECEIVE RECEPTION SIGNAL — T4

T5

YES

$V_C \geq V_D \times 0.2$ ?

NO

T6

HAS REFERENCE PERIOD PASSED ?

NO

UPDATE POSITION — T8

YES

T7

FOR ALL POSITIONS ?

NO

YES

END

FIG. 8

**FIG. 9**

| ELEMENT | TRANS-MISSION | PEELING DETECTION | | MEASUREMENT PERIOD | OPERATION MODE | DISPLAY IMAGE |
|---|---|---|---|---|---|---|
| END | | | | | START | |
| TRANSMIT | Ttx1 | | | ← SENSOR S32 | | |
| RECEIVE | | Tac1 | Th1 | ← NO PEELING DETECTION | PEELING DETECTION | START |
| TRANSMIT | Ttx2 | | | ← SENSOR S01 | | |
| RECEIVE | | Tac2 | Th2 | ← NO PEELING DETECTION | | |
| TRANSMIT | Ttx3 | | | ← SENSOR S64 | | |
| RECEIVE | | Tac3 | Th3 | ← NO PEELING DETECTION | | |
| TRANSMIT | Ttx1 | | | | | |
| RECEIVE | | Tac1 | Th1 | Trx1 | NORMAL (FIRST FRAME) | START |
| TRANSMIT | Ttx61 | | | | | |
| RECEIVE | | Tac61 | Th61 | Trx61 | | |
| TRANSMIT | Ttx1 | | | | | |
| RECEIVE | | Tac1 | Th1 | Trx1 | NORMAL (SECOND FRAME) | ONE FRAME |
| TRANSMIT | Ttx61 | | | | | |
| RECEIVE | | Tac61 | Th61 | Trx61 | | |
| TRANSMIT | Ttx1 | | | | | |
| RECEIVE | | Tac1 | Th1 | Trx1 | NORMAL (THIRD FRAME) | TWO FRAMES |
| TRANSMIT | Ttx2 | | | | | |
| RECEIVE | | Tac2 | Th2 | ← FIRST PEELING DETECTION | | |
| TRANSMIT | Ttx2 | | | | PEELING DETECTION + WARNING | TWO FRAMES + WARNING PROCESS |
| RECEIVE | | Tac2 | Th2 | ← SECOND PEELING DETECTION | | |
| TRANSMIT | Ttx2 | | | | | |
| RECEIVE | | Tac2 | Th2 | ← COMPLETION OF PEELING MODIFICATION | | |
| TRANSMIT | Ttx1 | | | | | |
| RECEIVE | | Tac1 | Th1 | Trx1 | NORMAL (THIRD FRAME) | TWO FRAMES |
| TRANSMIT | Ttx61 | | | | | |
| RECEIVE | | Tac61 | Th61 | Trx61 | | |

TIME

# FIG.10

| ELEMENT | TRANS-MISSION | PEELING DETECTION | | MEASUREMENT PERIOD | | OPERATION MODE | DISPLAY IMAGE |
|---|---|---|---|---|---|---|---|

TIME

| TRANSMIT | Ttx1 | | | | | START | |
| RECEIVE | | Tac1 | Th1 | | | | |
| | | | | Trx1 | | NORMAL (FOURTH FRAME) | THREE FRAMES |
| TRANSMIT | Ttx61 | | | | | | |
| RECEIVE | | Tac61 | Th61 | | | | |
| | | | | Trx61 | | | |
| TRANSMIT | Ttx1 | | | | | | |
| RECEIVE | | Tac1 | Th1 | | | | |
| | | | | Trx1 | | NORMAL (FIFTH FRAME) | FOUR FRAMES |
| TRANSMIT | Ttx2 | | | | | | |
| RECEIVE | | Tac2 | Th2 | | | | |
| | | | | Trx2 | | | |
| TRANSMIT | Ttx3 | | | | | | |
| RECEIVE | | Tac3 | Th3 | | ← FIRST PEELING DETECTION | | |
| TRANSMIT | Ttx3 | | | | | | |
| RECEIVE | | Tac3 | Th3 | | ← SECOND PEELING DETECTION | PEELING DETECTION + WARNING | FOUR FRAMES + WARNING PROCESS |
| | | | | | ← UNDER PEELING | | |
| TRANSMIT | Ttx3 | | | | END OF PEELING | | |
| RECEIVE | | Tac3 | Th3 | | ← DETECTION | | |
| END | | | | | ← END OF OPERATION | WARNING | |

# FIG.11

```
        ( MEASURE )
             │
   ┌───────────────────┐
   │     ESTABLISH     │─ W1
   │  MEASUREMENT MODE │
   └───────────────────┘
             │
   ┌───────────────────┐
   │  SELECT POSITION  │─ W2
   └───────────────────┘
             │
   ┌───────────────────┐
   │     GENERATE      │─ W3
   │ TRANSMISSION SIGNAL│
   └───────────────────┘
             │
   ┌───────────────────┐
   │     RECEIVE       │─ W4
   │  RECEPTION SIGNAL │
   └───────────────────┘
             │
   ┌───────────────────┐
   │ MAXIMUM VALUE OF  │─ W5
   │ RECEPTION VOLTAGE │
   └───────────────────┘
             │
   ┌───────────────────┐
   │     REGISTER      │─ W6
   │  REFERENCE VALUE  │
   └───────────────────┘
             │
   ┌───────────────────┐
   │     MEASURE       │─ W7
   │   ELAPSED TIME    │
   └───────────────────┘
             │
   ┌───────────────────┐
   │     REGISTER      │─ W8
   │ REFERENCE PERIOD  │
   └───────────────────┘
             │
        (   END   )
```

# FIG.12

TRANSMISSION
SIGNAL

ELECTRODE OF
ELEMENT

Th

0
Ttx
Tds
Tac
Tde

FIG.13

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | JP 2013 055978 A (SEIKO EPSON CORP) 28 March 2013 (2013-03-28)<br><br>* abstract *<br>* paragraph [0036] *<br>* figures 5, 6 *<br>----- | 1-4, 8-11,13, 15 | INV.<br>G01N29/04<br>G01N29/44 |
| X | EP 0 713 102 A1 (ADVANCED TECH LAB [US]) 22 May 1996 (1996-05-22)<br>* abstract *<br>* column 2, line 1 - line 5 *<br>* column 7, line 13 - line 28 *<br>* figure 7C *<br>----- | 1-15 | |

TECHNICAL FIELDS
SEARCHED      (IPC)

G01N
A61B

The present search report has been drawn up for all claims

| Place of search | Date of completion of the search | Examiner |
|---|---|---|
| Munich | 14 July 2016 | Rouault, Patrick |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 16 15 9345

This annex lists the patent family members relating to the patent documents cited in the above-mentioned European search report.
The members are as contained in the European Patent Office EDP file on
The European Patent Office is in no way liable for these particulars which are merely given for the purpose of information.

14-07-2016

| Patent document cited in search report | | Publication date | Patent family member(s) | | Publication date |
|---|---|---|---|---|---|
| JP 2013055978 | A | 28-03-2013 | JP | 5807459 B2 | 10-11-2015 |
| | | | JP | 2013055978 A | 28-03-2013 |
| EP 0713102 | A1 | 22-05-1996 | EP | 0713102 A1 | 22-05-1996 |
| | | | JP | H08238243 A | 17-09-1996 |
| | | | US | 5517994 A | 21-05-1996 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2013055978 A